# EUROPEAN PATENT SPECIFICATION

(11) **EP 1 994 906 B1**
(45) Date of publication and mention of the grant of the patent: **11.07.2012**
(21) Application number: 08007462.8
(22) Date of filing: 16.04.2008
(51) Int. Cl.: A61C 1/00, A61B 18/20, B23K 26/00, H01S 3/00

(54) **Laser system for hard body tissue ablation**
Lasersystem zur Ablation von festem Körpergewebe
Système laser pour ablation de tissu corporel dur

(30) Priority: 19.05.2007 EP 07010010
(43) Date of publication of application: 26.11.2008
(73) Proprietor: Fotona d.d., 1210 Ljubljana (SI)
(72) Inventor: Marincek, Marko, 1000 Ljubljana (SI); Lukac, Matjaz, 1000 Ljubljana (SI)
(74) Representative: Riedel, Peter

(56) References cited:
- WO-A-98/22185
- WO-A-99/38572
- WO-A-02/067802
- US-A- 5 269 778
- US-A1- 2003 226 831

## Description

The invention relates to a laser system with the features of the preamble of claim 1.

In the field of dentistry or the like, lasers are used for removal of hard body tissues such as dental enamel, dentine or bone material. The material removal in hard tissue ablation is based on a pronounced absorption of the laser in water; despite the minimal water contents or presence of water in hard body tissue, this enables a satisfactory material removal. The laser absorption leads to local heating with sudden water evaporation that, like a micro explosion, causes material removal.

The solid-state lasers that are typically used in the field of hard tissue ablation are operated in pulsed operation as a result of their system requirements in order to avoid overheating of the laser rod. At the same time, the pulsed operation contributes to heat being generated at the treatment location only for a very short time period and within a locally limited area. However, not only the aforementioned sudden water evaporation is generated by means of the temporally limited pulse length of an individual pulse but also an undesirable heating of the surrounding tissue is caused. Moreover, at the beginning of an individual pulse a small cloud of water vapor and removed particles is produced that shields the treatment location with regard to the temporally following section of the individual pulse and therefore reduces its effectiveness. For avoiding the aforementioned disadvantages, it is therefore desirable to use laser pulses with short pulse length, low energy, and short pulse periods as well as high repetition rate. Such a laser is known for example from US 2005/0033388 A1 and WO 02/067802 A1, with a Er:YAG laser having a pulse length of 5 to 500 fs (femtoseconds) with a pulse repetition rate of 50 kHz to 1 kHz, the latter corresponding to a pulse period of 20 µs to 1.000 µs (microseconds).

However, such an operating scheme will reduce the efficiency in other ways. A laser rod generates a laser beam only above a certain energy threshold that must be overcome by pumping, for example, by means of a flashlamp. At very short pulses of low energy, a significant portion of the pumping energy is required for overcoming this energy threshold before a usable laser energy is even made available. Therefore, according to a generally accepted teaching among persons skilled in the art, short pulses of low energy and high repetition rate have a bad efficiency and therefore provide minimal processing speed.

As a compromise, in the dental operating methods according to the prior art, as known for example from US 2003/0158544 A1, individual pulses with a pulse length of approximately 25 µs (microseconds) to 150 µs and a pulse period of approximately 16 ms (milliseconds) are used. The above described disadvantageous effects of heating the surroundings and shielding are however overcome only to an unsatisfactorily degree. The efficiency and obtainable treatment speed are minimal.

WO 99/38572 discloses a dual mode laser system for both skin tissue ablation and blood coagulation. The disclosed laser system comprises an Er:YAG laser, being alternatively operated with ablation pulses and coagulation pulse sequences. The disclosed coagulation pulse sequence has pulse spacing of 100 µs. However, this only applies to the coagulation mode and not the ablation mode. No details for the pulse spacing in ablation mode are given. The pulse length is 500 µs for ablation mode and 150 µs for coagulation mode.

The invention has the object to further develop a laser system of the aforementioned kind in such a way that its efficiency is improved.

This object is solved by a laser system having the features of claim 1.

A laser system for hard body tissue ablation is proposed, comprising a pumped laser, wherein the laser system is adapted to be operated in pulsed operation with several individual pulses of a temporally limited pulse length and wherein the individual pulses follow one another in a temporal pulse period, and are separated by a temporal pulse spacing. Here, the temporal pulse spacing is defined as the temporal difference between the end of one single pulse and the beginning of the next single pulse. The pumped laser has an inversion population remaining time being the time within which in the absence of pumping the remaining inversion population of the laser energy status is reduced by 90%, i.e. to 10% of the initial value. The pulse spacing is in the range of ≧ 50 µs, in partitular ≧ 80 µs, and less than the inversion population remaining time.

It is important to note that the inversion population remaining time is not always equal to the spontaneous decay time of the upper laser level. For example, in laser materials with high concentration of laser active atoms or ions (such as for example Er:YAG), or with appropriately chosen additional dopants (such as for example the Cr ions in Er:Cr:YSGG), the inversion population decay process may due to the energy up-conversion processes among interacting atoms or ions not be exponential, and subsequently the remaining time can be significantly longer than the spontaneous decay time. The inversion population time may in such cases vary with the inversion population and thus can be determined only approximately.

In a preferred further embodiment, the laser is an Er:YAG laser with a inversion population remaining time of ≤ 300 µs, wherein the temporal pulse spacing is ≤ 300 µs.

In a preferred further embodiment, the laser is an Er:YSGG or Er:Cr:YSGG laser with an inversion population remaining time of ≤ 3200 µs, wherein the temporal pulse spacing is ≤ 3200 µs.

In a preferred further embodiment, the laser is a solid-state laser with an inversion population remaining time of ≤ 200 µs, wherein the temporal pulse spacing is ≤ 200 µs.

With these time values, the invention deviates from the afore described teachings of the prior art and is based on the following recognition:

When an ablative laser light pulse is directed unto the hard tissue, an ablation of the tissue starts that leads to the emission of ablated particles above the hard tissue surface, forming a debris cloud. The debris cloud does not develop instantaneously. Particles begin to be emitted after some delay following the onset of a laser pulse, after which they spread at a certain speed and within certain spatial angle above the ablated tissue surface. So in the beginning the emitted particles are close to the surface, and at longer times the particles are well above the surface. The debris cloud interferes with the laser beam, resulting in laser light scattering. The undesired scattered portion of the laser beam is present to a significant extent only at the later time steps of the single laser pulse.

From the scattering viewpoint, the temporal pulse spacing between two subsequent single pulses should be longer than the time the debris cloud needs to settle down, the longer the better. This way there is no debris cloud remaining from the previous pulse. In particular, when between the end of one single pulse and the beginning of the next single pulse there remains sufficient time being greater that the cloud decay time of approximately 90 microseconds, any subsequent laser pulse will not encounter a debris cloud remaining from the preceding laser pulse.

However, from the viewpoint of laser efficiency it is advantageous not to use as long temporal pulse spacing as possible. This is because there is some inversion population of the laser energy status remaining after the end of the laser pulse. When a laser material is supplied with energy by pumping, the individual atoms are successively moved into the higher laser-enabling energy state. A significant share of the atoms remains at this higher energy state for a short period of time even after termination of the pumping process and even after termination of the laser emission. This period of time is limited by the inversion population remaining time, being the time within which in the absence of pumping the remaining inversion population of the laser energy status is reduced to 10% of the initial value. In case pumping for the second pulse starts early enough the threshold is reduced as the laser has been already pre-pumped from the previous pump pulse. From this viewpoint, the temporal pulse spacing should be shorter than the inversion population remaining time, i.e. the time within which in the absence of pumping the remaining inversion population of the laser energy status is reduced to 10%. The shortening of the pulse spacing in accordance with the present invention utilizes this effect in that after termination of a very short individual pulse and after completion of the very short temporal pulse spacing within the inversion population remaining time, there is still residual energy in the laser material that is available for the subsequent individual pulse.

So, a compromise is found according to the invention, where the temporal pulse spacing should be longer than the cloud decay time and shorter than the inversion population remaining time as follows: The residual laser energy is found to be useful to a technical extent for temporal pulse spacing ≤ to the inversion population remaining time. For suitable pulse lengths between 10 and 120 microseconds the cloud decay time is approximately on the order of 50 microseconds, so in the inventive combination the pulse spacing is between including 50 microseconds, in particular 80 microseconds and including the inversion population remaining time.

Contrary to the prior art prejudice, the laser can be operated with the afore defined short temporal pulse spacing, and in consequence with short pulse lengths being shorter than the pulse period, at low energy and at high efficiency so that a high treatment speed is enabled. The pulse period and thus the temporal pulse spacing between two individual pulses is large enough so that a debris cloud of removed material, water, and water vapor can escape from the beam path. The pulse length of the individual pulses is short enough that the shielding effect of the water vapor generation caused in the first time period of the individual pulse is of reduced importance or is even negligible during the subsequent second time period of the individual pulse. The impairment of the laser beam by the removed material is minimized in the second period of the individual pulse; the absorption of the laser light is reduced. The scattering of the beam is minimized so that the removal precision is improved. The heat load of the tissue surrounding the treatment location is minimized.

The pulse length is in the range of ≥ 10 µs and ≤ 120 µs. For this range, it is important to note that scattering of the light in the debris cloud represents a problem only when the cloud is high enough above the surface so that it can scatter the light of the laser beam considerably away from the original laser beam size. Since typical beam sizes are within 0.1 and 2 mm, scattering becomes a serious problem when the cloud reaches a height approximately 2 mm or higher. This happens approximately within 90 to 110 microseconds from the laser pulse onset. It therefore has been found, that with laser pulse durations of approximately 120 microseconds or shorter, the effect of scattering is almost non-existent, compared to the case when pulse duration of approximately 400 microseconds is used.

From the scattering viewpoint the pulse duration should therefore be equal to or shorter than 100 microseconds, the shorter the better. However, from the technical considerations (achievable pump powers with diodes that cannot pump enough energy within short times, or when flashlamp pumping is used, exponentially decreasing flashlamp lifetimes at shorter pulse durations) it is desirable to have long pulse lengths. Therefore a suitable compromise is to apply pump pulses with duration ≤ 120 microseconds and ≥ 10 microseconds.

However, even at this range of pulse lengths the laser efficiency of lasers such as Er:YAG, Er:YSGG or Er:Cr:YSGG is reduced as their operating efficiency is better in case longer pulse durations with higher energy outputs and lower repetition rates are used. However, by choosing the inventive train of pulses with temporal pulse spacings within the a.m. range, some of the efficiency is gained, that is lost by using shorter pulse durations resp. shorter pulse lengths. So, it is the combination of both pulse spacing and pulse duration ranges, that makes laser efficiency high enough even at the reduced light scattering.

In a preferred further embodiment, the individual pulses are combined to pulse sets that follow one another in a temporal set period wherein the pulse sets each comprise at least three individual pulses. Expediently, the pulse sets each have maximally 20 individual pulses, preferably however eight to twelve and in particular ten individual pulses. The temporal set period is preferably ≤ 50 ms, advantageously ≤ 30 ms and in particular approximately 20 ms. In the aforementioned embodiment of the method, the individual pulses as they are known in the prior art are at least partially replaced by the inventive pulse sets. Maintaining the aforementioned upper limit of the number of individual pulses per pulse set avoids overheating of the laser rod. Between the individual pulse sets there is enough time for cooling of the laser rod. The aforementioned minimum number of individual pulses per pulse set leads to an effective utilization of the residual energy that remains in the laser material after pumping so that the arrangement as a whole can be operated with high efficiency.

One embodiment of the invention will be explained in the following with the aid of the drawing in more detail. It is shown in:
- Fig. 1: a schematic illustration of a debris cloud generation during the course of a single laser pulse resulting in a scattering of the laser beam;
- Fig. 2: a diagrammatic illustration of the temporal debris cloud development close to the treated surface;
- Fig. 3: a diagrammatic illustration of the temporal debris cloud development at a greater distance to the treated surface compared to Fig. 2;
- Fig. 4: a diagrammatic illustration of the debris cloud time delay dependence on the distance from the ablated surface;
- Fig. 5: in a diagrammatic illustration the temporal course of pulse sets according to the present invention;
- Fig. 6: an enlarged diagrammatic illustration of a detail of a pulse set according to Fig. 5 with the temporal course of the individual pulses;
- Fig. 7: a measuring diagram of the actual pulse course according to Fig. 6 for explaining the utilization of the energy that is stored within the laser rod.

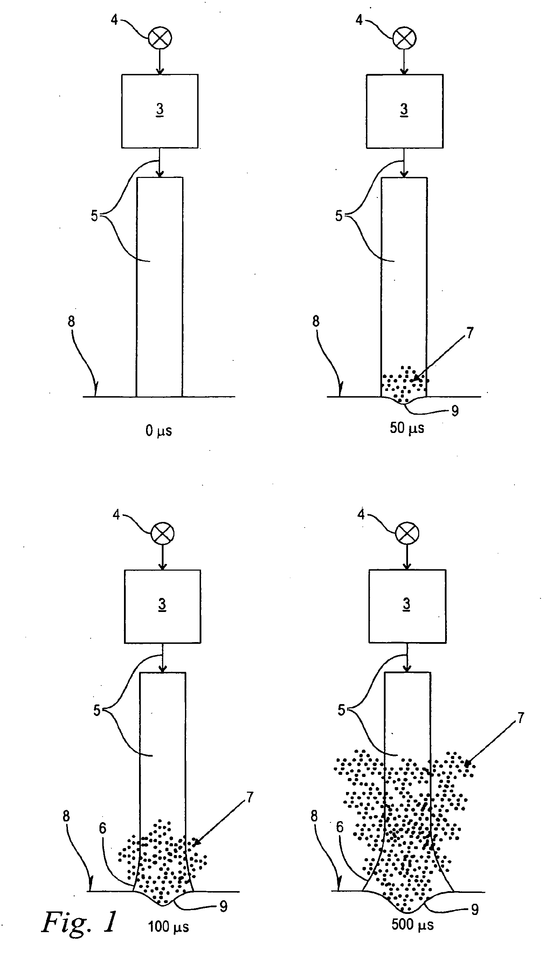

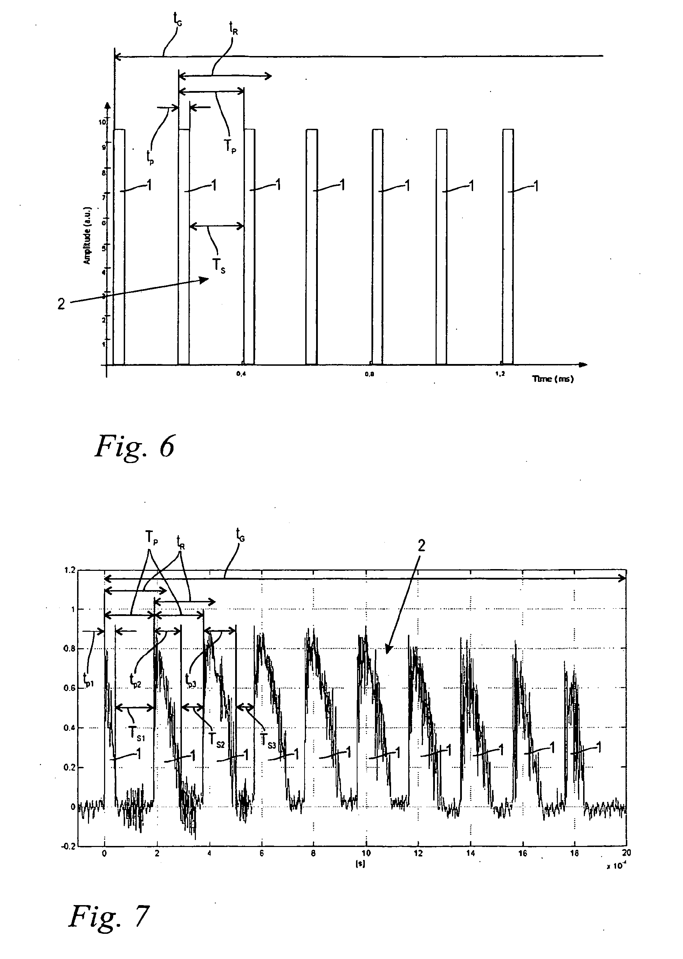

Fig. 1 shows a schematic illustration of a debris cloud 7 generation during the course of a single laser pulse at four different points of time, namely at the beginning of the single laser pulse at 0 µs, followed by subsequent time steps of 50 µs, 100 µs and 500 µs. A laser system comprises a laser 3 being a solid state laser, and being pumped by a flashlamp 4. The laser is a solid-state laser with a inversion population remaining time t_{R} of ≤ 200 µs, as illustrated in fig. 6, 7. The inversion population remaining time t_{R} is the time within which in the absence of pumping the remaining inversion population of the laser energy status is reduced by 90%. The laser is preferably an Er:YAG with an inversion population remaining time t_{R} of ≤ 300 µs, or an Er:YSGG (or Er:Cr:YSGG) laser with an inversion population remaining time t_{R} of ≤ 3200 µs (fig. 7). However, other solid state lasers or any other type of lasers such as liquid, diode, gas or fiber lasers can be used.

During pumping by the flashlamp 4, the laser 3 generates a pulsed laser beam 5, each pulse of the laser beam 5 corresponding to the flashlamp pulse. The pulsed laser beam 5 is directed to a treated surface 8 of hard body tissue like dental enamel, dentin or bone material. The laser beam 5 is schematically depicted as an arrow close to the laser 3. However, in practical use the laser has a certain diameter in the range of 0.1 to 2.0 mm, as shown close to the treated surface 8. Note that other pumping mechanisms, such as but not limited to diode pumping, instead of pumping with flashlamps may apply.

When the ablative laser light pulse is directed unto the hard tissue, an ablation of the tissue starts, forming an ablation area 9, and leading to the emission of ablated particles above the hard tissue surface 8, forming a debris cloud 7. The debris cloud 7 does not develop instantaneously, as can be seen in fig. 1 for the time step of 0 µs. Particles begin to be emitted after some delay following the onset of the laser pulse, after which they spread at a certain speed and within certain spatial angle above the ablated tissue surface. So in the beginning the emitted particles are close to the surface, and at longer times the particles are well above the surface, as can be seen in fig. 1 for the time steps of 50 µs, 100 µs and 500 µs. The debris cloud 7 interferes with the laser beam 5, resulting in laser light scattering and a scattered portion 6 of the laser beam 5. The undesired scattered portion 6 is present to a significant extent only at the later time steps of the single laser pulse, as can be seen e.g. for the time step of 500 µs.

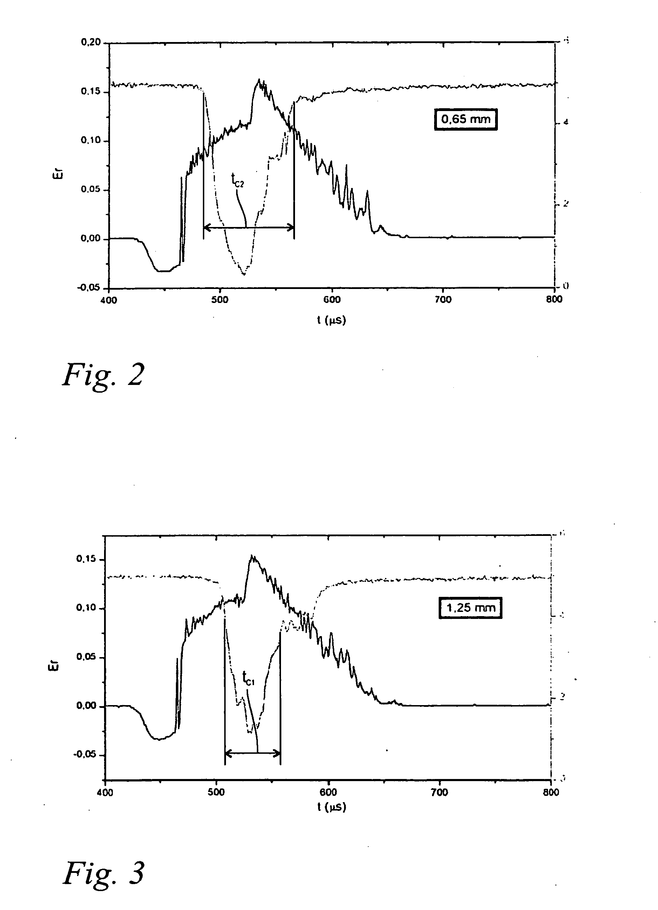

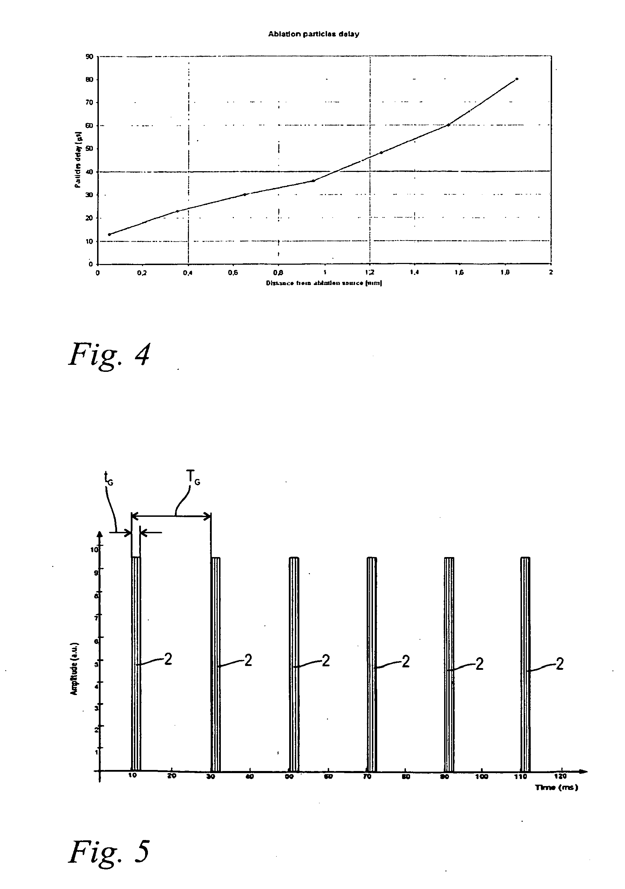

As an example, Figures 2 and 3 show the cloud development at the distances 0.65 mm and 1.25 mm from the treated surface 8 (Fig. 1) correspondingly. In the figures 2 and 3 the upsided curve represents the laser pulse temporal shape and the downsided curve represents the amount of scattered light at a particular spatial distance from the ablated surface.
As can be seen, the cloud development, measured by the level of light scattering, occurs later at larger distances from the surface 8 (fig. 1). It can be seen from the scattered light curves, that the debris cloud 7 (fig. 1) has typical cloud decay times t_{C1}, t_{C2} of approximately 50 µs and 80 µs respectively. Within the cloud decay times t_{C1}, t_{C2} the debris cloud 7 has settled down to an extent, that it does not disturb the laser beam 5 (fig. 1) significantly, and that it does not generate a significant scattered portion 6 of the laser beam 5 (fig. 1). Pulse spacings T_{S}, as described infra in connection with fig. 5 to 7, are therefore chosen to be equal to or longer than the cloud decay times t_{C1}, t_{C2}, i.e. ≥ 50 µs, and in particular ≥ 80 µs, in order to allow time for the debris cloud 7 (fig. 1) to settle down between individual pulses.

Fig. 4 shows the dependence of the time delay of the cloud development on the distance from the treated surface 8. It is important to note that scattering of the light in the debris cloud 7 (fig. 1) represents a problem only when the cloud is high enough above the surface so that it can scatter the light of the laser beam 5 (fig. 1) considerably away from the original laser beam size. Since typical beam sizes are within 0.1 and 2 mm of diameter, scattering becomes a serious problem when the cloud reaches a height of approximately 2 mm or higher. This happens (see Fig. 4) approximately within 90 to 110 microseconds from the laser pulse onset. It therefore has been found, that with laser pulse durations of approximately 120 microseconds or shorter, the effect of scattering is almost non-existent, compared to the case when pulse duration of approximately 400 microseconds is used.

Referring now simultaneously to fig. 1 to 7, the inventive laser system is adapted to be operated for hard body tissue ablation, the laser 3 being operated in pulsed operation wherein individual pulses 1 (fig. 6) of the laser 3 or of a laser beam generated by the laser 3 are combined to pulse sets 2 as explained infra in connection with Figs. 6 and 7.

Fig. 5 shows in a schematic diagram the temporal course of the pulse sets 2 according to the invention. In this connection, the course of the amplitude of the pulse sets 2 is illustrated as a function of time. The pulse sets 2 follow one another in a temporal set period T_{G}. The temporal set period T_{G} is expediently ≤ 50 ms, advantageously ≤ 30 ms, and is in the illustrated embodiment of the inventive method approximately 20 ms. The individual pulse sets 2 have a temporal set length t_{G} of, for example, approximately 2 ms. Depending on the number of individual pulses 1 provided infra the value of the temporal set length t_{G} can vary.

Fig. 6 shows an enlarged detail illustration of the diagram according to Fig. 5 in the area of an individual pulse set 2. Each pulse set 2 has at least three and maximally 20 individual pulses 1, respectively; preferably, each pulse set 2 has eight to twelve individual pulses 1 and in the illustrated embodiment according to Fig. 7 there are ten individual pulses 1 of which, for ease of illustration, only seven individual pulses 1 are illustrated in Fig. 6. The individual pulses 1 have a temporal pulse length tp and follow one another in a temporal pulse period T_{P}, the temporal pulse period T_{P} being the time period from the beginning of one single pulse 1 to the beginning of the next, subsequent pulse 1. The individual pulses 1 follow one another with a temporal pulse spacing Tₛ, the temporal pulse spacing Tₛ being the temporal difference between the end of one single pulse 1 and the beginning of the next single pulse 1. For generating the individual pulses 1 of the laser beam, the laser 3 is pumped by means of the flashlamp 4 (fig. 1) in pulsed operation. The temporal course of the flashlamp pulses corresponds with regard to the pulse length tₚ, the pulse spacing T_{S}, the pulse period Tp, and the pulse set period T_{G} to the temporal course of the individual pulses 1 or of the pulse sets 2 of the laser beam. In Fig. 6, the amplitude of the laser beam or of its individual pulses 1 is schematically plotted as a function of time wherein the temporal course of the individual pulses 1, for ease of illustration, are shown as rectangular pulses. In practice, the pulse course deviates in accordance with the illustration of Fig. 7 from the schematically shown rectangular shape of Fig. 6.

The pulse spacing T_{S} is, according to the invention, in the range between 50 µs, in particular 80 µs, and the inversion population remaining time t_{R}. For the particular case of an Er:YAG laser the pulse spacing T_{S} is ≤ 300 µs. For the particular alternative case of an Er:YSGG laser the pulse spacing T_{S} is ≤ 3200 µs. Preferably, for a solid state laser the pulse spacing T_{S} is ≤ 200 µs. The pulse length tp is in the range of ≥ 10 µs and ≤ 120 µs, in particular ≤ 50 µs. The pulse period T_{P} is chosen as an example to be 200µs. However, different pulse periods T_{P} may be applied. With the pulse length tp being in the range of ≥ 10 µs and ≤ 120 µs, and the sum of one pulse length tp and one pulse spacing T_{S} being equal to one pulse period T_{P}, the actual pulse spacings T_{S} are in the range of ≥ 80 µs and ≤ 190 µs.

Fig. 7 shows a measuring diagram of the course of an actual laser pulse wherein an Er:YAG laser was pumped with flash pulses of constant pulse length tp and constant pulse period Tp of 200 µs in accordance with the illustration of Fig. 6. The illustrated pulse set 2 has a total of ten individual pulses 1; in this connection, the amplitude of the generating laser beam is illustrated as a function of time. It can be seen that the first individual pulse has approximately a triangular course with a pulse length tₚ₁ of approximately 50 µs. After the first pulse a pulse spacing Tₛ₁ of approximately 150 µs has lapsed, the second individual pulse 1 with a pulse length tₚ₂ of approximately 100 µs, slightly increased in comparison to the pulse length of the first individual pulse 1, follows wherein the second individual pulse 1 relative to the first individual pulse 1 has a more "filled-out" course with a higher total energy quantity. The second pulse 1 with the pulse length tₚ₂ is followed by a pulse spacing T_{S2} of approximately 100 µs. For an unchanged energy input by means of pulsed pumping in accordance with the course of Fig. 6, approximately after the third individual pulse 1 a pulse length tₚ₃ of approximately 120 µs will result, followed by a pulse spacing Tₛ₃ of approximately 80 µs. The third individual pulse 1 has, in comparison to the first two individual pulses 1, a more filled-out pulse course so that the energy of the individual pulse 1 is further increased relative to the preceding first two individual pulses 1, in spite of the pump energy remaining the same. The short pulse spacings Tₛ₁, Tₛ₂, Tₛ₃ of approximately 150 µs to 80 µs are well below the inversion population remaining time t_{R} of ≤ 300 µs of the Er:YAG laser as used here. In consequence, during the pumping process in particular of the first two individual pulses 1, a portion of the pumped energy is saved in the laser rod and is not completely given off in the form of laser energy. As a result of the short pulse spacing Tₛ, a part of the saved energy is available for generating the energy yield in the case of the subsequent individual pulses 1.

On the other hand, as can be further derived from Fig. 7, the shown pulse spacings Tₛ are ≥ 50 µs and even ≥ 80 µs. Between the end of one single pulse 1 and the beginning of the next single pulse 1 there remains some time in the range between including 80 µs and including 150 µs, which is in the same order or even greater than the cloud decay time t_{C} of approximately 50 µs or 80 µs (fig. 2, 3). So any subsequent laser pulse will not encounter a debris cloud 7 (fig. 1) remaining from the preceding laser pulse 1.

Toward the end of the pulse set 2, i.e., upon completion of, for example, ten individual pulses 1 with a temporal set length t_{G} of approximately 2 ms the energy of individual pulses 1 is reduced as a result of thermal effects. After lapse of the set period T_{G} (Fig. 5) and the start of a new pulse set 2, the complete energy schematic according to Fig. 7 is available again, however.

## Claims

1. Laser system for hard body tissue ablation, comprising a pumped laser, wherein the laser system is adapted to be operated in pulsed operation with several individual pulses (1) of a temporally limited pulse length (tₚ) and wherein the individual pulses (1) follow one another with a temporal pulse spacing (Tₛ),
**characterized in that** the pumped laser has a inversion population remaining time (t_{R}), the inversion population remaining time (t_{R}) being the time within which in the absence of pumping the remaining inversion population of the laser energy status is reduced by 90% and that the pulse spacing (Tₛ) is in the range of ≥ 50 µs and ≤ to the inversion population remaining time (t_{R}), and that the pulse length (tₚ) is in the range of ≥ 10 µs and ≤ 120 µs.

2. Laser system according to claim 1,
**characterized in that** the pulse spacing (Tₛ) is ≥ 80 µs.

3. Laser system according to claim 1 or 2,
**characterized in that** the laser is an Er:YAG laser with a inversion population remaining time (t_{R}) of ≤ 300 µs, and that the pulse spacing (Tₛ) is ≤ 300 µs.

4. Laser system according to claim 1 or 2,
**characterized in that** the laser is an Er:YSGG or an Er:Cr:YSGG laser with a inversion population remaining time (t_{R}) of ≤ 3200 µs, and that the pulse spacing (Tₛ) is
≤ 3200 µs.

5. Laser system according to one of the claims 1 to 4,
**characterized in that** the laser is a solid-state laser with a inversion population remaining time (t_{R}) of ≤ 200 µs, and that the pulse spacing (Tₛ) is ≤ 200 µs.

6. Laser system according to one of the claims 1 to 5,
**characterized in that** the individual pulses (1) are combined to pulse sets (2) which follow one another in a temporal set period (T_{G}) wherein the pulse sets (2) each comprise at least three individual pulses (1).

7. Laser system according to claim 6,
**characterized in that** the pulse sets (2) each comprise maximally twenty individual pulses (1).

8. Laser system according to claim 6,
**characterized in that** the pulse sets (2) each comprise eight to twelve and in particular ten individual pulses (1).

9. Laser system according to one of the claims 6 to 8,
**characterized in that** the temporal set period (T_{G}) is ≤ 50 ms, advantageously ≤ 30 ms, and in particular approximately 20 ms.

## Patentansprüche

1. Lasersystem für die Ablation von hartem Körpergewebe, umfassend einen gepumpten Laser, wobei das Lasersystem für einen Impulsbetrieb mit mehreren Einzelimpulsen (1) einer zeitlich begrenzten Impulslänge (tₚ) ausgelegt ist, und wobei die Einzelimpulse (1) mit einem zeitlichen Pulsabstand (Tₛ) aufeinander folgen,
**dadurch gekennzeichnet, dass** der gepumpte Laser eine Zeit verbleibender Besetzungsinversion (t_{R}) aufweist, wobei die Zeit verbleibender Besetzungsinversion (t_{R}) diejenige Zeit ist, innerhalb derer in Abwesenheit von Pumpen die verbleibende Besetzungsinversion des Laserenergiezustandes um 90% reduziert ist, dass der zeitliche Pulsabstand (Ts) in einem Bereich von ≥ 50 µs und ≤ der Zeit verbleibender Besetzungsinversion (t_{R}) liegt, und dass die Impulslänge (tₚ) in einem Bereich von > 10 µs und ≤ 120 µs liegt.

2. Lasersystem nach Anspruch 1,
**dadurch gekennzeichnet, dass** der Pulsabstand (Tₛ) ≥ 80 µs ist.

3. Lasersystem nach Anspruch 1 oder 2,
**dadurch gekennzeichnet, dass** der Laser ein Er:YAG Laser mit einer Zeit verbleibender Besetzungsinversion (t_{R}) von ≤ 300 µs ist, und dass der Pulsabstand (Tₛ) ≤ 300 µs ist.

4. Lasersystem nach Anspruch 1 oder 2,
**dadurch gekennzeichnet, dass** der Laser ein Er:YSGG oder ein Er:CR:YSGG Laser mit einer Zeit verbleibender Besetzungsinversion (t_{R}) von ≤ 3200 µs ist, und dass der Pulsabstand (Tₛ) ≤ 3200 µs ist.

5. Lasersystem nach einem der Ansprüche 1 bis 4,
**dadurch gekennzeichnet, dass** der Laser ein Festkörperlaser mit einer Zeit verbleibender Besetzungsinversion (t_{R}) von ≤ 200 µs ist, und dass der Pulsabstand (Tₛ) ≤ 200 µs ist.

6. Lasersystem nach einem der Ansprüche 1 bis 5,
**dadurch gekennzeichnet, dass** die Einzelimpulse (1) zu Impulsgruppen (2) zusammengefasst sind, die in einer zeitlichen Gruppenperiode (T_{G}) aufeinander folgen, wobei die Impulsgruppen (2) jeweils mindestens drei Einzelimpulse (1) aufweisen.

7. Lasersystem nach Anspruch 6,
**dadurch gekennzeichnet, dass** die Impulsgruppen (2) jeweils maximal zwanzig Einzelimpulse (1) aufweisen.

8. Lasersystem nach Anspruch 6,
**dadurch gekennzeichnet, dass** die Impulsgruppen (2) jeweils acht bis zwölf und insbesondere zehn Einzelimpulse (1) aufweisen.

9. Lasersystem nach einem der Ansprüche 6 bis 8,
**dadurch gekennzeichnet, dass** die zeitliche Gruppenperiode (T_{G}) ≤ 50 ms ist, vorteilhaft ≤ 30 ms ist, und insbesondere etwa 20 ms beträgt.

## Revendications

1. Système laser pour ablation de tissus corporels durs, comprenant un laser pompé, dans lequel le système laser est apte à être utilisé en fonctionnement pulsé avec plusieurs impulsions individuelles (1) d'une longueur d'impulsion limitée dans le temps (tₚ) et dans lequel les impulsions individuelles (1) se suivent à un espacement d'impulsion dans le temps (Tₛ),
**caractérisé en ce que** le laser pompé a un temps de population d'inversion restante (t_{R}), le temps de population d'inversion restante (t_{R}) étant le temps au cours duquel, en l'absence de pompage, la population d'inversion restante du statut d'énergie laser est réduite de 90 %, et **en ce que** l'espacement d'impulsion (Tₛ) est dans la plage de ≥ 50 µs et ≤ au temps de population d'inversion restante (t_{R}), et **en ce que** la longueur d'impulsion (tₚ) est dans la plage de ≥ 10 µs et ≤ 120 µs.

2. Système laser selon la revendication 1,
**caractérisé en ce que** l'espacement d'impulsion (Tₛ) est ≥ 80 µs.

3. Système laser selon la revendication 1 ou 2,
**caractérisé en ce que** le laser est un laser Er:YAG avec un temps de population d'inversion restante (t_{R}) ≤ 300 µs, et **en ce que** l'espacement d'impulsion (Tₛ) est ≤ 300 µs.

4. Système laser selon la revendication 1 ou 2,
**caractérisé en ce que** le laser est un laser Er:YSGG ou Er:Cr:YSGG avec un temps de population d'inversion restante (t_{R}) ≤ 3200 µs, et **en ce que** l'espacement d'impulsion (Tₛ) est ≤ 3200 µs.

5. Système laser selon l'une quelconque des revendications 1 à 4,
**caractérisé en ce que** le laser est un laser à semiconducteurs avec un temps de population d'inversion restante (t_{R}) ≤ 200 µs, et **en ce que** l'espacement d'impulsion (Tₛ) est ≤ 200 µs.

6. Système laser selon l'une quelconque des revendications 1 à 5,
**caractérisé en ce que** les impulsions individuelles (1) sont combinées à des ensembles d'impulsions (2) qui se suivent dans une période d'ensemble dans le temps (T_{G}), dans lequel les ensembles d'impulsions (2) comprennent chacun au moins trois impulsions individuelles (1).

7. Système laser selon la revendication 6,
**caractérisé en ce que** les ensembles d'impulsions (2) comprennent chacun un maximum de vingt impulsions individuelles (1).

8. Système laser selon la revendication 6,
**caractérisé en ce que** les ensembles d'impulsions (2) comprennent chacun de huit à douze et en particulier dix impulsions individuelles (1).

9. Système laser selon l'une quelconque des revendications 6 à 8,
**caractérisé en ce que** la période d'ensemble dans le temps (T_{G}) est ≤ 50 ms, de manière avantageuse ≤ 30 ms, et en particulier environ 20 ms.
